# EUROPEAN PATENT APPLICATION

(11) **EP 4 104 829 A1**
(43) Date of publication of application: **21.12.2022**
(21) Application number: 21382535.9
(22) Date of filing: 17.06.2021
(51) Int. Cl.: A61K 31/20

(54) **LAURYL GALLATE FOR USE AS ANTIVIRAL AGENT**

(71) Applicant: Consejo Superior de Investigaciones Científicas (CSIC), 28006 Madrid (ES)
(72) Inventor: LOPEZ CARRASCOSA, Angel Luis, 28049 Madrid (ES); SOBRINO CASTELLO, Francisco, 28049 Madrid (ES); TORRES LORITE, Elisa, 28049 Madrid (ES); CAÑAS ARRANZ, Rodrigo, 28049 Madrid (ES); SÁIZ ZALABARDO, Margarita, 28049 Madrid (ES); DE LEÓN VALDÉS, Patricia, 28049 Madrid (ES); BUSTOS SANCHEZ, Maria Jose, 28049 Madrid (ES)
(74) Representative: Pons

(57) **Abstract**

The invention relates to a new use of Lauryl Gallate (hereafter, LG) as an antiviral agent in the treatment and/or prevention of coronavirus infections and more particularly to human coronavirus infections, such as HCoV-229E or SARS-CoV-2 infections.

## Description

The invention relates to a new use of Lauryl Gallate (hereafter, LG) as an antiviral agent in the treatment and/or prevention of coronavirus infections and more particularly to human coronavirus infections.

### BACKGROUND ART

Gallic acid derivatives, formerly used for their antioxidant or antitumoral activities, have been also described for their antiviral activity as possible tools for the prevention of DNA and RNA virus dissemination:
A) Several gallic acid esters have been used as antioxidants under many different conditions, including as part of a pharmaceutical composition or formulation. Alkyl (propyl, octyl and lauryl) esters of gallic acid, known as antioxidant food additives (E-310, E-311 and E-312, respectively), were also reported to inhibit the proliferation of tumoral cell lines (Calcabrini A., et al. Carcinogenesis 2006, 27:1699-1712) by triggering the apoptosis of the cell (Serrano A. et al. Biochem. Biophys. 1998, 350, 49-54) and the concomitant inhibition of protein tyrosine kinases (Palacios C. et al. J. Enzyme Inhib. 2001, 16, 527-533), thus providing a possible protective effect against carcinogenesis.
B) Several reports in the literature illustrate the antiviral activity of alkyl gallates, for example, with anticancer agents showing promise against orthopoxviruses and perhaps other viruses as well (Kane C.J. et al. Biosci Rep 1988; 8:95-102.), or more specifically, the effect of octyl and lauryl gallate on many virus infections produced by enveloped DNA and RNA virus species (Uozaki M. et al. Antiviral Res 2007; 73:85-91). The effect of LG might be mediated by its interaction with phospholipids in biological membranes (Jurak M. et al. Biochim Biophys Acta 2016; 1858:1821-1832.), with surfactant effects destabilizing the cellular and viral envelopes.

In the case of LG, we previously found a consistent antiviral activity in cells infected by foot-and-mouth disease virus (FMDV), in concentrations of the drug that were non-cytotoxic, reducing the virus productivity up to 2 log.u. The inhibition of FMDV production is specific for LG among the different alkyl gallates derivatives, resulting OG (octyl gallate), PG (propyl gallate) and GA (gallic acid) totally ineffective against FMDV at non cytotoxic concentrations. In addition, a reduction of mortality, clinical signs and viral RNA copies in different tissues at day 2 post-infection was found in FMDV-infected C57 mice that had received one daily dose of LG (100 mg/Kg) starting 24 h before virus inoculation (de Leon A. L. et al. Front. Microbiol. 2019, 10 (1853), 1-11 and WO2020099696).

Cell-directed antiviral compounds against coronaviruses, mainly SARS-CoV-2, would have numerous advantages and this has been approached by different authors in the clinical context of drug repurposing (Gordon et al. Nature 2020. 583: 459-468). In the case of the current COVID-19 situation, such advantages include the presumable activity of LG against several virus variants, the reduction of virus spread and the possibility of becoming a complementary tool in settings where vaccination is administered. Indeed, Umar et al. (Umar, H.I., et al. J Genet Eng Biotechnol 2021, 19, 16) have identified LG, among other compounds, as a potential anti SARS-CoV-2 drug based on docking experiments (binding affinity). These predictive results differ from the experimental results we present here in that the binding energy of LG and different SARS-CoV-2 proteins is lower than epicatechin gallate and gallic acid, compounds that inhibit SARS-CoV-2 multiplication in VERO E6 cells to a much lesser extent than LG.

### SUMMARY OF THE INVENTION

The inventors have found that LG acts as inhibitor of cell culture infection with coronaviruses such as HCoV-229E (alphacoronavirus) and SARS-CoV-2 (betacoronavirus) at non cytotoxic concentrations. A robust virus inhibition of 1-2 log.u. is produced when LG is added a few hours before, after or simultaneously to the infection. Also, the inventors have studied LG presentations, their stability at room temperature, solubility and shelf life and also methods for drug delivery into the hots (mainly humans), for quick and safe administration.

The invention is directed to the field of drugs affecting cellular processes, instead of targeting viral proteins or functions, to control virus dissemination.

Then, in a first aspect, the present invention relates to Lauryl Gallate, its, tautomers and/or solvates, for use in the prevention and/or the treatment of coronavirus infection.

Lauryl Gallate (LG) has the next formula:

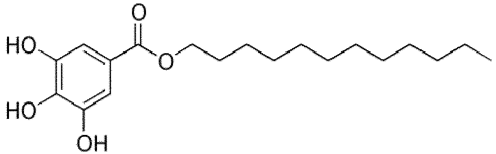

LG is a known compound that can be obtained synthetically by direct esterification reaction of gallic acid with lauryl alcohol (dodecanol) (J. Am. Chem. Soc., 1947, 69, 2003-2005). However, they are also available commercially.

In a preferred embodiment, the present invention refers to Lauryl Gallate, its tautomers and/or solvates, for use in the prevention and/or the treatment of coronavirus infection, more preferably, human coronavirus infection and, even more preferably, HCoV-229E or SARS-CoV-2 infection.

Unless otherwise stated, the compounds used in the invention are intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in ¹³C or ¹⁴C or a nitrogen atom enriched in ¹⁵N fall within the scope of this invention.

The term "pharmaceutically acceptable" relates to molecular entities and compositions that are physiologically tolerable and do not typically produce an allergic reaction or a similar unfavourable reaction, such as gastric upset, dizziness and similar side effects, when administered to a subject. Preferably, the term "pharmaceutically acceptable" means approved by a regulatory agency of a federal or state government or collected in the US Pharmacopoeia or other generally recognised pharmacopeia for use in animals and, more particularly, in humans.

The compounds used in the invention may be in crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

"Tautomers" are understood to be the two isomers that differ only in the position of a functional group because between the two forms there is a chemical balance in which a migration of a group or atom occurs.

The term "treatment or prevention" as used herein, unless otherwise indicated, relates to reversing, alleviating and inhibiting the progress of, or preventing the disorder or condition to which it applies in such terms, one or more symptoms of such disorder or condition.

Other aspect of the present invention relates to a pharmaceutical composition comprising LG, its tautomers and/or solvates, as defined above for use in the prevention and/or the treatment of coronavirus infection, more preferably, human coronavirus infection and, even more preferably, HCoV-229E or SARS-CoV-2 infection.

The term "pharmaceutical composition" means the composition that includes the LG, its tautomers and/or solvates, and at least one excipient, adjuvant and/or pharmaceutically acceptable carrier.

The term "excipients, adjuvants and/or carriers" relates to molecular entities or substances through which the active ingredient is administered. Such pharmaceutical excipients, adjuvants or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes. Suitable pharmaceutical excipients and carriers are described in "Remington's Pharmaceutical Sciences" by E.W. Martin.

The pharmaceutical composition is administrated in a therapeutically effective amount and can be administered by any suitable administration route, for example, oral, topical or intraperitoneal.

For its application in therapy, the compound for use of the invention will preferably be in a pharmaceutically acceptable or substantially pure form, that is, the compound has a pharmaceutically acceptable level of purity excluding pharmaceutically acceptable excipients and not including material considered toxic at normal dosage levels. The purity levels for the compound are preferably above 50%, more preferably above 70%, more preferably above 90%. In a preferred embodiment, they are above 95%.

As used herein, the term "therapeutically effective amount" means the necessary amount of a compound for the treatment or prevention of the disease, disorder or condition to be effective. It will also depend on the subject to be treated, the severity of the illness suffered by said subject, the chosen form of administration, etc. For this reason, the doses mentioned in this invention should be considered only as guidelines for the person skilled in the art, and he should adjust the doses according to the variables mentioned above. However, LG, its tautomers and/or solvates can be administered in mice intraperitoneally once or twice a day, in a typical daily amount between 100 and 200 mg/kg body weight/day, preferably 100 mg/kg body mass daily.

LG, its tautomers and/or solvates as well as the compositions containing them can be used in conjunction with other additional antiviral drugs, preferably useful in the prevention and / or treatment of coronavirus infections. Said additional drugs may form part of the same composition or, alternatively, may be provided in the form of a separate composition for simultaneous or sequential administration to the composition comprising LG, its tautomers and/or solvates.

In another aspect, the invention relates to a method for the prevention and/or treatment of coronavirus infection in a subject, comprising the administration to said subject of a therapeutically effective amount of LG, its tautomers and/or solvates. More preferably the coronavirus infection is a human coronavirus infection and even more preferably is HCoV-229E or SARS-CoV-2 infection.

In another aspect, the invention relates to the use of LG, its tautomers and/or solvates for the preparation of a medicament for prevention and/or treatment of coronavirus infection. More preferably the coronavirus infection is a human coronavirus infection and even more preferably is HCoV-229E or SARS-CoV-2 infection.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration and are not intended to be limiting of the present invention.

### DESCRIPTION OF THE DRAWINGS

**FIG. 1****.** Direct virotoxicity of LG on HCoV-229E-GFP and SARS-CoV-2.
**FIG. 2****.** "In vitro" Inhibition of HCoV-229E-GFP and SARS-CoV-2 by LG.
**FIG. 3****.** Effect of time of addition of LG in HCoV-229E-GFP infection.
**FIG. 4****.** Specificity of LG inhibition for HCoV-229E-GFP
**FIG. 5****.** Specificity of LG inhibition for SARS-CoV-2

### Examples

### 1. Cytotoxicity of LG in Huh7, VeroE6 and Caco2 cell lines.

Cell viability determinations were performed by the MTT assays, as previously described (Hurtado C., et al. Antiviral Therapy. 2008, 13:909-91). Briefly, cell monolayers were grown on 96-well plates before the addition of the corresponding concentration of lauryl gallate (LG, Sigma), using six wells for each dose. After 24h of incubation medium was replaced with MTT-containing culture medium, incubated 2h and lysed with SDS-containing lysis solution. Absorbance at 550 nm was determined after 15 min of incubation, and the average values (from 6 wells) obtained were substracted from the background levels (in the absence of cells) and compared with the data scored in the absence of the drug (100% viability). From the viability curves of each cell (not shown) we calculate the CC50 value (concentration of LG yielding a 50% of cell viability) (Table 1).

From these results, working concentrations of 1 to 40 µM LG were chosen to perform experiments under non-cytotoxic conditions.

**Table 1:**

| Cell line | **Organism** | **Virus** | **CC50 (µM)** |
|---|---|---|---|
| Huh-7 | Human | HCoV-229E-GFP | 83.12 |
| Vero-E6 | Primate | SARS-CoV-2 | 90.09 |
| Caco-2 | Human | SARS-CoV-2 | 97.07 |

### 2. Direct virotoxicity of LG on HCoV-229E-GFP and SARS-CoV-2.

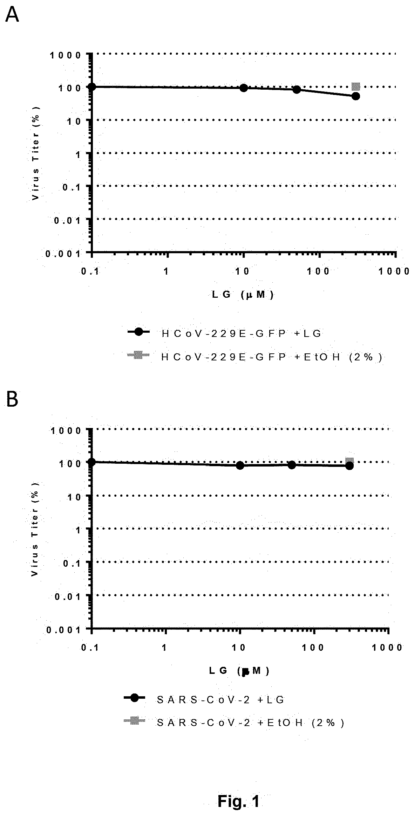

To determine a possible direct virucidal effect of LG on the virus particle, suspensions of HCoV-229E-GFP or SARS-CoV-2 MAD-6 (10⁵ pfu) in 0.9 ml of culture medium were added with 0.1 ml of LG dilutions to obtain the indicated LG concentration (from 0 to 300 µM); controls with viruses incubated in 2% ethanol in the absence of the drug were incubated in parallel. After 1h incubation at room temperature, ten-fold dilutions of each sample in culture medium were immediately prepared and titrated by plaque assay on virus-sensitive Huh-7 (for HCoV-229E-GFP) or Vero-E6 (for SARS-CoV-2) cell monolayers. Virus titers were compared in duplicate samples with those obtained in virus samples incubated in the absence of the drug. No direct toxicity was observed in HCoV-229E-GFP (Fig. 1A) or SARS-CoV-2 (Fig. 1B) samples when incubated with LG at the indicated range of concentrations.

### 3. Inhibition of HCoV-229E-GFP and SARS-CoV-2 by LG.

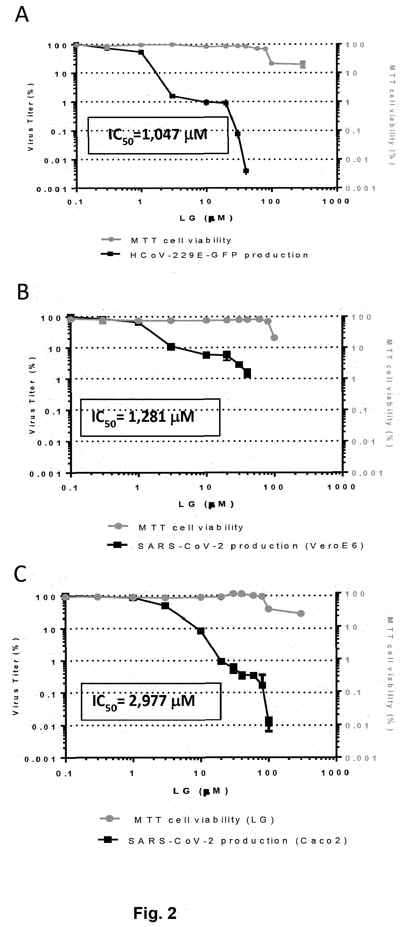

Huh-7 (for HCoV-229E-GFP) and Vero-E6 or Caco-2 (for SARS-CoV-2) cell monolayers were grown on 96 well plates and incubated for 1h with the indicated concentrations of LG (from 0,3 to 40 µM, Fig. 2). Stock solutions of lauryl gallate (LG), in 2% ethanol (a concentration previously shown to preserve more than 80% cell viability), were diluted in DMEM to obtain the working stocks. After incubation with LG, duplicate cultures were infected with HCoV-229E-GFP or SARS-CoV-2 (MAD-6) at a m.o.i. of 2 pfu/cell, in a reduced volume of medium containing the LG for 2 h, released from the virus inoculum and washed twice with medium, before the addition of drug-containing fresh medium (supplemented with 2% FCS). Cultures were then incubated for 24h. Total virus production was evaluated by plaque assay in Huh-7 (for HCoV-229E-GFP) or Vero-E6 (for SARS-CoV-2) cells.

As shown in Fig. 2A, a consistent decrease of more than 2 log.u. in the HCoV-229E-GFP yield was observed at non-toxic concentrations of LG (IC₅₀=1,047 µM). In the case of SARS-CoV-2, a similar inhibition range was obtained both in Vero-E6 (IC₅₀=1,281 µM, Fig. 2B) and in Caco-2 (IC₅₀=2,977 µM, Fig. 2C) cell lines.

### 4. Effect of time of addition of LG in HCoV-229E-GFP infection.

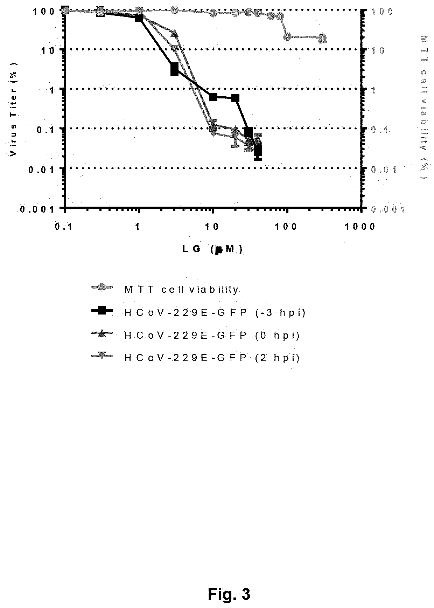

To assess the efficiency of LG under more restrictive conditions, a trial was done in which the drug was added at the time or after infection. Huh-7 cells were grown in 96-well plates and infected with HCoV-229E-GFP at a m.o.i. of 2 pfu/cell. After 2 h virus adsorption [0 hours post-infection, (0 hpi)], cultures were washed away twice from non-adsorbed virus and further incubated at 33ºC in culture medium. Different concentrations of LG (from 0,3 to 40 µM, Fig.3) were added to duplicate wells at three different times (-3, 0 and 2 hpi) and cultures were further incubated until 24 hpi, when they were collected and the virus produced quantified by plaque assay. Although the most efficient inhibition was found when the drug was added before infection (IC₅₀=1,235 µM), non-significant differences were obtained when added at the time (IC₅₀=1,481 µM) or after (IC₅₀=2,05 µM) infection (Fig.3).

### 5. Specificity of LG inhibition

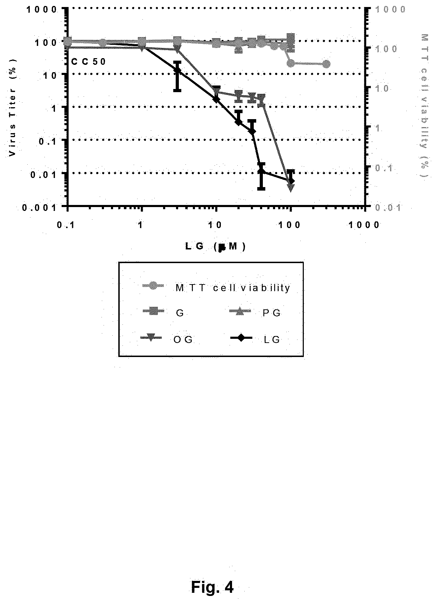

To determine the specificity of LG in the inhibition of HCoV-229E-GFP multiplication, the effect of several gallate esters (propyl-, octyl- and lauryl-gallate) including gallic acid (Uozaki M, et al. Antiviral Res. 2007 73:85-91), was analyzed in HCoV-229E-GFP - infected Huh7 cells. As shown in Fig. 4, gallic acid (G) and propyl-gallate (PG) were unable to produce virus inhibition at non-toxic concentrations (up to 100 µM), while both octyl-(OG) and lauryl-gallate reduced up to 2 log.u. the HCoV-229E-GFP infection, in a range of concentrations (1 to 30 µM) which still maintained cell viability. Although both compounds reduced virus yields, a substantial difference was that HCoV-229E-GFP infection was more resistant to OG (IC₅₀=6,210 µM) than to LG (IC₅₀=1,215 µM),

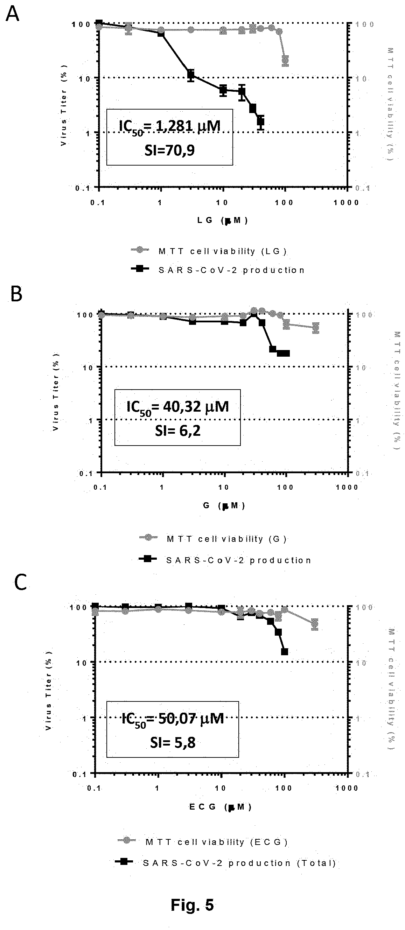

In the case of SARS-CoV-2, gallic acid and epicatechin gallate (ECG) were tested in order to determine the specificity of LG inhibition. The magnitude of the LG inhibition of SARS-CoV-2 relative to those of gallic acid and epicatechin gallate would be relevant (chemical specificity), as the binding inferred for these two molecules to SARS-CoV-2 proteins is predicted to be higher than that for LG (Umar, H.I., et al. J Genet Eng Biotechnol 2021, 19, 16). As shown in Fig. 5 the antiviral capacity of both gallic acid (IC₅₀=40,32 µM, SI=6,2) and epicatechin gallate (IC₅₀=50,07 µM, SI=5,8) was very low, compared to that of LG (IC₅₀=1,215 µM, SI=70,9).

## Claims

1. Lauryl Gallate, its tautomers and/or solvates, for use in the prevention and/or the treatment coronavirus infection.

2. Lauryl Gallate, its tautomers and/or solvates, for use, according to claim 1, wherein the coronavirus is a human coronavirus.

3. Lauryl Gallate, its tautomers and/or solvates, for use, according to claim 2, wherein the human coronavirus is HCoV-229E or SARS-CoV-2.

4. Pharmaceutical composition that comprises Lauryl Gallate, its tautomers and/or solvates for use in the prevention and/or the treatment of coronavirus infection.

5. Pharmaceutical composition for use, according to claim 4, wherein the coronavirus is a human coronavirus.

6. Pharmaceutical composition for use, according to claim 5, wherein the human coronavirus is HCoV-229E or SARS-CoV-2.

7. Pharmaceutical composition for use, according to any of claims 4-6, wherein said composition is administered in combination with another antiviral drug.

8. Pharmaceutical composition for use, according to any of claims 4-7, wherein the composition is adapted for its oral, topical or intraperitoneal administration.
